# EUROPÄISCHE PATENTSCHRIFT

(11) **EP 0 685 070 B1**
(45) Veröffentlichungstag und Bekanntmachung des Hinweises auf die Patenterteilung: **03.09.2003**
(21) Anmeldenummer: 95905571.6
(22) Anmeldetag: 21.12.1994
(51) Int. Cl.: G01N 33/53, G01N 33/68, G01N 33/58, G01N 33/569, G01N 33/74, G01N 33/543, G01N 33/531

(54) **ACYLIERTE PROTEINAGGREGATE UND DEREN VERWENDUNG ZUR ENTSTÖRUNG VON IMMUNOASSAYS**
ACYLATED PROTEIN AGGREGATES AND THEIR USE IN SUPPRESSING INTERFERENCE IN IMMUNOASSAYS
AGREGATS DE PROTEINES ACYLES ET LEUR UTILISATION POUR EVITER LES PERTURBATIONS DANS DES DOSAGES IMMUNOLOGIQUES

(30) Priorität: 21.12.1993 DE 4343479
(43) Veröffentlichungstag der Anmeldung: 06.12.1995
(73) Patentinhaber: Roche Diagnostics GmbH, 68298 Mannheim (DE)
(72) Erfinder: SCHLIEPER, Dittmar, D-82362 Weilheim (DE); SCHMID, Franz, D-86911 Diessen (DE); KAUFMANN, Martin, D-82362 Weilheim (DE)
(86) Internationale Anmeldenummer: EP9404264
(87) Internationale Veröffentlichungsnummer: WO95017668

(56) Entgegenhaltungen:
- EP-A- 0 260 903
- EP-A- 0 269 092
- EP-A- 0 347 138
- EP-A- 0 525 916
- GB-A- 1 505 400
- J. IMMUNOL. METHODS (1985), 85(2), 409-19 CODEN: JIMMBG;ISSN: 0022-1759, 1985 Kenna, J. G. et al 'Methods for reducing non-specific antibody binding in enzyme- linked immunosorbent assays'
- J. IMMUNOL. METHODS (1991), 143(2), 159-65 CODEN: JIMMBG;ISSN: 0022-1759, 1991 Guidry, Albert J. et al 'Prevention of nonspecific binding of immunoglobulin to Staphylococcus aureus protein A in ELISA assays'

## Beschreibung

Die Erfindung betrifft acylierte Proteinaggregate, deren Herstellung und deren Einsatz in einem Entstörmittel und in einem Bindereagenz fiir immunologische Teste und deren Verwendung zur Entstörung von Immunoassays sowie ein entsprechendes immunologisches Nachweisverfahren.

Immunologische Nachweismethoden haben in den letzten Jahren eine große Bedeutung erlangt. Mit ihnen kann die Gegenwart von Arzneimitteln, Hormonen, Proteinen und insbesondere infektiösen Organismen in biologischen Proben schnell und genau nachgewiesen werden. Bei allen immunologischen Nachweismethoden kommt es zu einer spezifischen Bindungsreaktion zwischen einem ersten spezifischen Bindungspanner, der Substanz, die nachgewiesen werden soll ( "Analyt" oder "Ligand") und einem zweiten spezifischen Bindungspartner, der spezifisch mit dem Liganden reagiert oder ihn bindet. Ligand und spezifischer Ligandbindungspartner, die sogenannten Partner eines spezifischen Bindungspaares, bilden dabei ein spezifisches Bindungspaar, im allgemeinen ein Komplex zwischen einem Antigen und einem Antikörper oder Antikörperfragment. Dabei können mehr als ein Ligand oder ein Bindungspartner in jeder Reaktion miteinander reagieren. Diese spezifischen Bindereaktionen werden auf verschiedene Weise detektiert. Im allgemeinen ist ein Teilnehmer der spezifischen Bindereaktion markiert. Übliche Markierungsmethoden sind Radioisotope, Chromogene, Fluorogene oder Enzymmarkierung. Bei heterogenen Immunoassays ist einer der Bindungspartner an eine Festphase immobilisiert.

Ein schwerwiegendes Problem bei Immunoassays ist, daß zwischen spezifischen Bindungspartnern des Immunoassays und der Probe, der in der Probe enthaltenen zusätzlichen Bestandteile und gegebenenfalls der Festphase unerwünschte Wechselwirkungen und unspezifische Bindereaktionen erfolgen können. Derartige Wechselwirkungen bewirken im allgemeinen eine Erhöhung des Hintergrundsignals und auch eine stärkere Streuung der Signale und damit eine verringerte Sensitivität und Spezifität des betreffenden Testes. Durch unspezifische Wechselwirkung mit dem markierten Bindungspartner können auch falsch-positive Messungen resultieren, das heißt, es wird fälschlicherweise die Anwesenheit eines Analyten auch bei dessen Abwesenheit gemessen.

Es wurden verschiedene Versuche unternommen, diese unspezifischen Wechselwirkungen in Immunoassays zu reduzieren. Seit langem ist bekannt, daß unterschiedliche Kohlenhydratkomponenten und unterschiedliche Proteine, Proteingemische oder Proteinfraktionen sowie deren Hydrolysate unspezifische Wechselwirkungen zwischen den Testkomponenten und dem Analyten in Immunoassays reduzieren können (beispielsweise Robertson et al., Journal of Immun.Meth. 26, 1985, 195, EP-A-260903, US-A-4,931,385). Der Einsatz von Proteinrohfraktionen und Rohhydrolysaten hat den Nachteil, daß durch die darin enthaltenen Verunreinigungen wiederum andere Störungen des Tests ausgelöst werden können. Enzymatisch produzierte Hydrolysate können zudem mit den bei der Herstellung verwendeten Proteasen kontaminiert sein und weisen in der Regel keine einheitliche Qualität auf, da sich die Spaltung nur schwer steuern läßt. Proteasekontaminationen können Testkomponenten angreifen und schon in geringen Mengen zur Beeinträchtigung der Testfunktionen und Lagerstabilität führen.

EP-A-0 331 068 beschreibt den Einsatz von polymerisiertem Immunoglobulinen (IgG) zur Reduktion spezifischer Störfaktoren wie z.B. Rheumafaktoren. Es lassen sich damit aber nicht unspezifische Wechselwirkungen, insbesondere solche der markierten Bindungspartner mit Analyten oder mit der Festphase zufriedenstellend ausschalten. Ferner ist die Gewinnung von humanem oder tierischem IgG aufwendig und teuer.

Zur Verringerung unspezifischer Wechselwirkungen in Immunoassays wurde auch der Einsatz von chemisch modifizierten Proteinen, insbesondere succinylierten Proteinen, beschrieben (US-A-5,051,356, EP-A 525916 7. Immunol Meth- 85:409-419(1985). Doch wird insbesondere bei Analysen auf hochmolekulare Analyte, zum Beispiel virale Antigene trotz sehr hoher Konzentrationen an Proteinentstörsubstanzen gemäß dem Stand der Technik keine zufriedenstellende Entstorung des Testes gewährleistet.

Aufgabe der Erfindung war es daher, neue Entstörsubstanzen bzw. neue Entstörmittel zur Verfügung zu stellen, die eine bessere Entstörung bei Immunoassays als aus dem Stand der Technik bekannt bewirken. Insbesondere sollten die Entstörsubstanzen einen niedereren Leerwert, eine Verringerung der Signalstreuung und die Vermeidung von falsch-positiven Analysenergebnissen insbesondere bei der Analyse von hochmolekularen Analyten bewirken.

Diese Aufgabe wird gelöst durch spezifisch acylierte Proteinaggregate.

Gegenstand der Erfindung sind Proteinaggregate als Entstörsubstanzen für Immunoassays die mit -CO-R Gruppen acyliert sind, wobei R einen verzweigten oder unverzweigten, C1-C4-Alkylrest darstellt, der mit Carboxy, Hydroxy, SO₃H oder PO₃H₂ substituiert sein kann.

Ein weiterer Gegenstand der Erfindung ist ein entsprechendes Entstörmittel für Immunoassays enthaltend eine proteinhaltige Entstörsubstanz und einen Puffer, dadurch gekennzeichnet, daß es ein oder mehrere der erfindungsgemäßen acylierten Proteinaggregate enthält.

Ein weiterer Gegenstand der Erfindung ist ein spezifisches Bindereagenz für Immunoassays enthaltend einen Partner eines spezifischen Bindungspaares, dadurch gekennzeichnet, daß es zusätzlich eine oder mehrere der erfindungsgemäßen Entstörsubstanzen oder Entstörmittel enthält.

Ein weiterer Gegenstand der Erfindung ist ein Verfahren zur Verminderung unspezifischer Wechselwirkungen in Immunoassays durch Inkontaktbringen der erfindungsgemäßen Entstörsubstanz oder des erfindungsgemäßen Entstormittels mit den bei einem Immunoassay verwendeten spezifischen Bindungspartnern eines spezifischen Bindungspaares.

Insbesondere ist Gegenstand der Erfindung ein Verfahren zur Bestimmung immunologischer Liganden in einer Probe unter Reduktion unspezifischer Wechselwirkungen durch
1) Kontaktieren der auf den Liganden zu untersuchenden Probe mit
   a) einer oder mehreren Entstörsubstanzen, oder einem oder mehreren Entstörmitteln enthaltend eine Entstörsubstanz und einen geeigneten Puffer und
   b) einem oder mehreren spezifischen Bindungspartnern von spezifischen Bindungspaaren, wobei mindestens ein Bindungspartner markiert ist und ein detektierbares Bindungspaar bildet.
2) Messung der Anwesenheit oder die Menge des markierten Bindungspaares oder des freien markierten Partners eines spezifischen Bindungspaares als Maß fiir die Anwesenheit oder Menge des Liganden in der Probe,
dadurch gekennzeichnet,
daß die Entstörsubstanz ein Proteinaggregat ist, das mit -CO-R Gruppen acyliert ist, wobei R einen verzweigten oder unverzweigten, C1-C4-Alkylrest, der mit Carboxy, Hydroxy, SO₃H oder PO₃H₂ substituiert sein kann, bedeutet.

Als Ligand dient die chemische oder biologische Substanz, die mit einem oder mehreren entsprechenden spezifischen Bindungspartner spezifisch zu einem Komplex reagiert wie zum Beispiel Proteine, Peptide, Kohlenhydrate, Toxine, Haptene, Arzneimittel, Viren, Pilze und Bakterien, Antikörper oder Bestandteile daraus u.a. Besonders geeignet ist die Erfindung fiir die Analyse von hochmolekularen Liganden wie zum Beispiel Viren, Virusmarker aber auch Hormone, insbesondere polyvalente Proteine wie HIV-Viren, prostataspezifisches Antigen (PSA), Thyreotropin (TSH), carcino embryonales Antigen (CEA), Hepatitis B-Viren (Hepatitis B surface antigen, HBs), A-Fetoprotein (AFP), Humanes Choriongonadotropin (HCG), lutenisierendes Hormon (LH) follikelstimulierendes Hormon (FSH), Prolactin, Ferritin, Insulin.

Als Probe dienen im allgemeinen Körperflüssigkeiten wie Blut, Serum oder Plasma, Speichel, Urin oder andere Körperflüssigkeiten.

Als spezifischer Bindungspartner kann jeder biologische oder chemische Bindungspartner dienen, der spezifisch mit einer anderen biologischen Substanz zu einem spezifischen Bindungspaar reagiert. Dazu zählen Antikörper, Antikörperfragmente, Antigene, Haptene, Hormone, Avidin, Biotin oder Derivate davon. Bevorzugt werden in der vorliegenden Erfindung als Partner eines spezifische Bindungspaares Antikörper oder Antikörperfragmente eingesetzt, die mit Antigenen spezifisch binden.

Mindestens einer der spezifischen Bindungspartner in einem Immunoassay ist markiert. Die Markierung kann direkt oder indirekt ein meßbares Signal liefern, zum Beispiel durch Radioaktivität, Chemilumineszenz, Phosphoreszenz, Fluoreszenz oder Elektrochemilumineszenz oder sichtbare Farbe. Der spezifische Bindungspartner kann auch indirekt detektierbar sein, zum Beispiel mit Enzymmarkierung, Biotin- oder Avidinmarkierung, die in einer oder mehreren Reaktionen teilnehmen, um eine detektierbare Substanz zu erzeugen. Bevorzugt wird Enzymmarkierung eingesetzt, insbesondere mit Peroxidase, Glucoseoxidase β-Galactosidase oder alkalischer Phosphatase. Eine weitere bevorzugte Markierung ist die Markierung mit einem chemolumineszierenden, insbesondere elektrochemolumineszierenden Molekül.

Die Erfindung ist dadurch gekennzeichnet, daß die erfindungsgemäßen Entstörsubstanzen mit -CO-R-Gruppen acylierte Proteinaggregate sind. Unter einem Proteinaggregat versteht man ein Aggregat, das aus gleichen oder verschiedenen definierten Proteinmonomeren zu einem höhermolekularen Partikel polymerisiert wurde. Definitionsgemäß wird dabei unter einem Proteinaggregat ein künstliches Partikel aus mindestens 2, bevorzugt 3 - 40.000. besonders bevorzugt 30 - 600 Proteinmonomeren, verstanden, die so fest aneinander gebunden sind, daß sie in wäßriger Lösung nicht in die Proteineinzelmoleküle zerfallen. Bevorzugt sind die Proteinaggregate wasserlöslich.

Proteine können thermisch oder chemisch polymerisiert oder aggregiert werden.

Bei der thermischen Polymerisierung finden sich Proteinmonomere unter Anwendung von höheren Temperaturen zu Aggregaten zusammen. Die thermische Polymerisierung von Proteinen ist am Beispiel von Albuminen in EP-A-269 092 beschrieben.

Die chemische Polymerisierung von Proteinmonomeren erfolgt mit nicht-proteinhaltigen homooder heterobifunktionellen Linkermolekülen. Diese Verfahren zur Vernetzung von Proteinen sind dem Fachmann bekannt und werden beispielsweise in GB-A 1505 400, EP-A-0 122 209 oder EP-A 269 092 beschrieben. Beispiele für die Vernetzung von Proteinmonomeren mit heterobifunktionellen Linkern sind die Reaktion mit Bis(maleinimido)-methylester, Dimethylsuberimidat, Disuccinimidyl-suberat, Glutardialdehyd, N-Succinimidyl-3-(2-pyridyldithio)propionat, N-5-Azido-2-nitrobenzoylsuccinimid, N-Succinimidyl(4-Jodacetyl)-aminobenzoat oder die Kombination von Maleinimidohexanoyl-N-Hydroxysuccinimidester (MHS) oder Maleinimido-benzoyl-NHS (MBS) und N-Succinimidyl-3-Acetyl-Thiopropionat (SATP). Beispiele für homobifunktionelle Linker sind zum Beispiel Diaminohexan, Carbodiimid und andere.

Bevorzugte Proteine sind Proteine mit einem Molekulargewicht höher 2 000, insbesondere höher 10 000. Albumine oder Ovalbumin sind besonders bevorzugt, besonders Serumalbumine, ganz besonders bevorzugt Rinderserumalbumin.

Bevorzugt werden in den erfindungsgemäßen Verfahren Proteinpolymere verwendet, die thermisch aggregiert wurden. Ganz besonders bevorzugt ist thermisch polymerisiertes Albumin, bevorzugt ein Serumalbumin, insbesondere Rinderserumalbumin ("Thermo-RSA"), das dann anschließend acyliert, insbesondere acetyliert oder succinyliert wird. Die Darstellung des nichtacylierten Thermo-Rinderserumalbumin ist in EP-A 269 092 beschrieben.

Vorteilhafterweise wird dabei die Polymerisation so durchgeführt und gesteuert, daß Polyproteinaggregatpartikel einer bestimmten möglichst einheitlichen Größe entstehen. Bevorzugt ist dabei eine Partikelgröße von 10 - 200 nm, ganz besonders vorteilhaft zwischen 20 und 50 nm. Dies entspricht einem Molekulargewicht von 240.000 Da - 2,2 x 10⁹ Da, bevorzugt 2,2 x 10⁶ - 35 x 10⁶ Da. Die Partikelgröße kann über bekannte Verfahren wie z.B. PCS (Photon Correlation Spectroscopy) bestimmt werden. Wenn nötig kann auch der fiir die Erfindung besonders geeignete Partikelgrößenbereich durch Gelfiltration von einem Rohpolymerisatgemisch abgetrennt werden, um eine besonders einheitliche Partikelgröße zu erhalten.

Die Proteinmonomere, die zur Polymerisierung eingesetzt werden, können dabei gleich oder verschieden sein. Bevorzugt werden einheitliche Proteinmonomere polymerisiert. Bevorzugt werden als Proteinmonomere Albuminmonomere eingesetzt. Als Albuminmonomere können alle tierischen oder menschlichen Albumine, insbesondere Serumalbumine eingesetzt werden. Ganz besonders für die Erfindung geeignet ist Rinderserumalbumin (RSA).

Die Proteinaggregate sind erfindungsgemäß mit -CO-R Gruppen acyliert, in denen R einen verzweigten oder unverzweigten C1-C4-Alkylrest darstellt, der mit Carboxy, Hydroxy, PO₃H₂ oder SO₃H substituiert sein kann. Besonders bevorzugt als Substituent ist die Carboxygruppe.

Dabei können die Acylgruppen schon in die Proteinmonomere oder erst nach Polymerisation der Proteinmonomere in die Proteinaggregate eingeführt werden. Acylierung von Proteinen erfolgt dabei nach bekannten Methoden, bevorzugt mit Acylanhydriden, oder mit Acyl-O-Succinimid. Als besonders vorteilhaft haben sich acetylierte und succinylierte Proteinaggregate, insbesondere Albuminaggregate erwiesen (R= Methyl bzw. -CH₂-CH₂-COOH). Dabei wird für die Acetylierung bevorzugt Essigsäure-O-Succinimid verwendet. Die Succinylierung erfolgt bevorzugt mit Bernsteinsäureanhydrid.

Bei der Acylierung werden im wesentlichen freie Aminogruppen (z.B. Lysinreste) des Proteinaggregates acyliert. Unter acylierten Proteinaggregaten wird verstanden, daß mindestens eine der vorhandenen freien Aminogruppen acyliert vorliegt. Bevorzugt wird aber eine möglichst vollständige Acylierung aller freien Aminogruppen.

Ein weiterer Gegenstand der Erfindung ist ein Entstörmittel fiir immunologische Teste, enthaltend einen Puffer für immunologische Teste und die erfindungsgemäße Entstörsubstanz. Dabei können als Puffer alle wäßrigen Puffer dienen, die in immunologischen Testen gebräuchlich sind, zum Beispiel Phosphat-, Glycin-HCI oder Glycin-NaOH, Acetat, Carbonat, Citrat-, oder organische Puffer wie z.B. Imidazol / HC; Triethanolamin; MES =(4-Morpholinoethansulfonsäure), TRIS = (TRIS(hydroxymethyl)-aminomethan), HEPES=(4-(2-Hydroxyethyl)-1-piperazinethan-sulfonsäure), MOPS (3-N-Morpholino-propan-sulfonsäure) und andere ähnliche Puffer. Der pH-Wert und, die Konzentration der Puffersalze richtet sich nach dem jeweiligen immunologischen Test, zum Beispiel unter anderem auch nach dem Enzym bei Enzymmarkierung. Übliche pH-Werte liegen zwischen 4 und 9. Übliche Pufferkonzentrationen liegen zwischen 1 mM und IM.

Die Konzentration an Entstörsubstanz richtet sich danach, mit welcher Menge an immunologischen Testkomponenten und den darin enthaltenen Störkomponenten das Entstörmittel zusammen gebracht werden soll. Für gebräuchliche Immunoassays sollte dabei die Konzentration an Entstörsubstanz im Entstörmittel so hoch sein, daß nach dem Zusammenbringen mit den immunologischen Testkomponenten eine Konzentration zwischen 1mg / ml und 50 mg /ml, bevorzugt 5 mg / ml bis 20 mg / ml resultiert. In Einzelfällen können aber auch bis zu 200 mg / ml erforderlich sein.

Zusätzlich können im erfindungsgemäßen Entstormittel noch weitere Zusätze wie Stabilisierungsmittel und ähnliches anwesend sein. Zum Einsatz in einem Immunoassay liegt das Entstörmittel vorteilhafterweise in wäßriger Pufferlösung vor. Zudem ist seine Anwendung als Imprägnierung eines porösen Trägermaterials (Vlies) z.B. auf einem Teststreifen und seine Lagerung in fester Form, beispielsweise als Lyophilisat möglich.

Ein weiterer Gegenstand der Erfindung ist ein spezifisches immunologisches Bindereagenz mit einem Partner eines spezifischen Bindungspaares, und der erfindungsgemäßen Entstörsubstanz oder Entstörmittel.

Das Bindereagenz gemäß der vorliegenden Erfindung wird so hergestellt, daß ein oder mehrere der spezifischen Bindungspartner eines Immunoassays und mindestens eine erfindungsgemäße Entstorsubstanz oder ein Entstörmittel gemischt werden. Gegebenenfalls können weitere Zusätze wie Stabilisierungsmittel oder Konservierungsmittel und ähnliches zugegeben werden. Die Menge des spezifischen Bindungspartners hängt von der immunologischen Testführung, der Menge des zu bindenden Bindungspartners, der Art der Markierung und anderen Faktoren ab. Im allgemeinen beträgt die Konzentration 1-20 µg / ml.

Die Menge an Entstörsubstanz richtet sich nach der Menge der immunologischen Testkomponenten und Störkomponenten, die im Bindereagenz enthalten sind und mit denen das Bindereagenz in Kontakt gebracht wird. Vorteilhafterweise sollte die Konzentration an Entstörsubstanz im Bindereagenz so hoch sein, daß nach dem Zusammenbringen mit den immunologischen Testkomponenten eine Gesamtkonzentration zwischen 1 und 50 mg / ml bevorzugt 5-20 mg / ml resultiert. Es können jedoch auch höhere Konzentrationen bis 200 mg / ml in Einzelfällen zu einer wirkungsvollen Entstorung notwendig sein.

Das spezifische Bindereagenz kann in jedem homogenen oder heterogenen Immunoassay verwendet werden, in dem ein spezifischer Bindungspartner für den Nachweis oder das Fehlen eines spezifisch bindenden Liganden nützlich ist. Beispiele dafür sind Sandwichassays, kompetitive Immunoassays und andere, dem Fachmann bekannte Immunoassays. Die Teste können in

Lösung oder auf festen Trägern durchgeführt werden.

Im allgemeinen wird das erfindungsgemäße Immunoassayverfahren so durchgeführt, daß eine einen Liganden enthaltende Probe mit einem spezifischen Bindungsreagenz gemäß der vorliegenden Erfindung in Lösung kontaktiert wird, so daß sich ein spezifischer Bindekomplex direkt oder indirekt zwischen dem Liganden und dem spezifischen Bindungspartner bildet. Möglich ist aber auch, daß der Ligand selbst als erfindungsgemäßes Bindereagenz vorliegt und mit einem spezifischen Bindungspartner oder mit einem weiteren erfindungsgemäßen Bindungsreagenz in Kontakt gebracht wird. Der Ligand und der Bindungspartner können direkt komplexieren. Der Bindungspartner ist dann spezifisch für den Liganden. Möglich ist aber auch, daß der Bindungspartner indirekt mit dem Liganden über eine oder mehrere spezifische Bindemoleküle komplexiert, die mit dem Liganden untereinander binden.

Wird das Verfahren auf festen Trägern, z.B. Tubes, Mikrotiterplatten oder einem Testträger als heterogener Immunoassay eingesetzt, kann auf dem Träger ein spezifischer Bindungspartner fiir den Liganden direkt immobilisiert sein. Bevorzugt ist aber, daß auf den Träger ein spezifischer Bindungspartner für den Ligandenbindungspartner immobilisiert ist. Ein bevorzugtes Beispiel dafür ist immobilisiertes Streptavidin als spezifisches Bindereagenz für biotinylierte Bindepartner. Die verschiedenen Variationen solcher heterogenen Immunoassays sind dem Fachmann bekannt.

Bei kompetitiven Immunoassays konkurrieren Ligand und ein markiertes Ligandanalogon um den unmarkierten Ligandenbindungspartner, der sich - bei heterogenen Immunoassays - über eine zweite Bindungsstelle bevorzugt eine spezifische Bindestelle wie Biotin) an die Festphase binden kann. Freie oder gebundene Ligandenanaloge werden durch ihre Markierung als Maß fiir die Anwesenheit oder Menge des zu bestimmenden Liganden gemessen.

Bei Sandwichimmunoassays bindet der Ligand mit einer ersten spezifischen Bindungsstelle an einen markierten Ligandenbindungspartner und mit der zweiten Bindungsstelle an einen unmarkierten, - bei heterogenen Immunoassays - mit einer weiteren spezifischen Bindungsstelle für die Festphase versehenen Ligandenbindungspartner. Es bildet sich ein Komplex zwischen Liganden, markierten und unmarkierten Bindungspartner, wobei sich der Komplex bei heterogenen Testen über den unmarkierten Bindungspartner an die Festphase bindet, und beispielsweise durch Waschen von dem freien markierten Bindungspartner getrennt werden kann.

Bestimmt werden freie oder gebundene markierte Ligandenbindungspartner als Maß für die Anwesenheit oder Menge des zu bestimmenden Liganden nach bekannten Methoden. Bei Enzymmarkierung beispielsweise wird zur markierten Spezies ein farbbildendes Enzymsubstrat zugegeben und die entstandene Farbe gemessen.

Mindestens einer der Partner eines spezifischen immunologischen Bindungspaares (Ligand, markiertes Ligandanalogon oder Ligandenbindungspartner) liegt als erfindungsgemäßes Bindungsreagenz zusammen mit der erfindungsgemäßen Entstörsubstanz zum Einsatz in einem Immunoassay vor. Vorteilhafterweise ist dies im allgemeinen ein Ligandenbindungspartner, insbesondere ein markierter Ligandenbindungspartner.

Ein weiterer Gegenstand der Erfindung ist die Verwendung einer erfindungsgemäßen Entstörsubstanz in Immunoassays.

Insbesondere ist Gegenstand der Erfindung die Verwendung einer erfindungsgemäßen Entstörsubstanz zur Reduktion unspezifischer Wechselwirkungen in Immunoassays.

Es hat sich gezeigt, daß die erfindungsgemäßen Entstörsubstanzen falsch-positive Signale von Negativproben beträchtlich reduzieren und damit auch das Leerwertsignal von positiven Proben. Ferner wird die Streuung des Leerwertes von positiven Proben und damit die Standardabweichung des Messwertes verkleinert. Dadurch wird auch der dynamische Messwertbereich ausgeweitet und die Messung insgesamt empfindlicher und genauer.

Ein weiterer Gegenstand der Erfindung ist ein Verfahren zur Herstellung der erfindungsgemäßen Entstörsubstanzen. Es ist dadurch gekennzeichnet, daß bevorzugt in einem ersten Schritt ein Protein, bevorzugt ein Albumin, insbesondere Rinderserumalbumin, durch chemische Aggregation mit bifunktionellen Linkern, bevorzugt durch thermische Aggregation aggregiert wird, bevorzugt auf eine Partikelgröße zwischen 10 und 200 nm, ganz besonders bevorzugt zwischen 20 und 50 nm. Die thermische Aggregation erfolgt bevorzugt bei einer Temperatur zwischen 50 und 100 °C, ganz besonders bevorzugt zwischen 60 und 80 °C in einem zweiten Schritt erfolgt dann die Acylierung mit einer -CO-R-Gruppe mit einem geeigneten Acylierungsmittel. Die Acylierung soll dabei bevorzugt vollständig verlaufen und kann über den Verbrauch an Acylierungsmittel über beispielsweise HPLC mitverfolgt werden.

Es ist jedoch auch möglich, das Verfahren in umgekehrter Reihenfolge durch Acylierung von Protein gemäß US-A-5,051,356 und anschließender thermischer oder chemischer Polymerisation des acylierten Proteins durchzuführen.

### Beispiel 1

Leerwertsenkende und entstörende Wirkung von acetyliertem Thermo-RSA

Ein Sandwichimmunoassay auf HBsAg (Hepatitis B Surface Antigen) wird mit dem Testsystem HBsAg Enzymuntest ® der Firma Boehringer Mannheim durchgeführt.
a) Test ohne acetyliertes Thermo-RSA:
   Inkubationspuffer mit Konjugat
      40 mmol Phosphatpuffer pH 7.0, Anti-HBsAg-Biotin (monoklonal Maus) > 240 ng / ml Pepton (Hydrolysat von Lactalbumin): 40 mg / ml
      Anti-HBsAg-POD (monoklonal Maus), POD (Peroxidase): 0,04 U / ml
   Substrat/Chromogen-Puffer:
      Phosphat/Citratpuffer 100 mmol/ l, pH 4,4;
      H₂O₂: 3,2 mmol/l;
      2,2' Azino-di [3-ethyl-benzthiazolin-sulfonsäure (6)]-diammoniumsalz (ABTS): 1,9 mmol / l

   Der Test wird auf einem Gerät ES 600 der Firma Boehringer Mannheim durchgeführt.
   100 µl Probe und 500 µl Inkubationslösung mit Konjugat werden in ein mit Streptavidin beschichtetes Röhrchen (Enzymuntest Firma Boehringer Mannheim) gegeben und inkubiert (180 min bei 37 ° C). Nichtfestphasengebundene, markierte Antikörper werden mit 200 µl Waschlösung aus dem Röhrchen entfernt.
   500 µl Substrat/Chromogenpufferlösung werden zugeben und die Farbentwicklung nach 60 min spektrophotometrisch bei 422 nm gemessen.
b)
   dem Inkubationspuffer werden in verschiedenen Versuchen "Entstörmittel" beigemischt:
   1. acetyliertes Thermo-RSA (thermisch aggregiertes Rinderserumalbumin) gemäß der Erfindung (2 mg / ml, Partikelgröße 30 nm)
   2. succinyliertes Thermo-RSA gemäß der Erfindung (2 mg / ml, Partikelgröße 30 nm)
   3. monomeres acetyliertes RSA gemäß Stand der Technik (2 mg / ml)
   4. monomeres succinyliertes RSA (2 mg / ml) gemäß Stand der Technik ( 2mg / ml)

Tabelle 1 zeigt die Messung verschiedener Proben ohne (gemäß Beispiel 1a) und mit verschiedenen Inkubationspufferzusätzen 1-4 (gemäß Beispiel 1b). Es zeigt sich eine deutliche Leerwertabsenkung der Testergebnisse mit den erfindungsgemäßen Entstörmitteln (gemäß Beispiel 1b, 1. und 2.) und eine Verringerung der Standardabweichung bei den Negativseren (NS) gegenüber den anderen Zusätzen gemäß Stand der Technik.

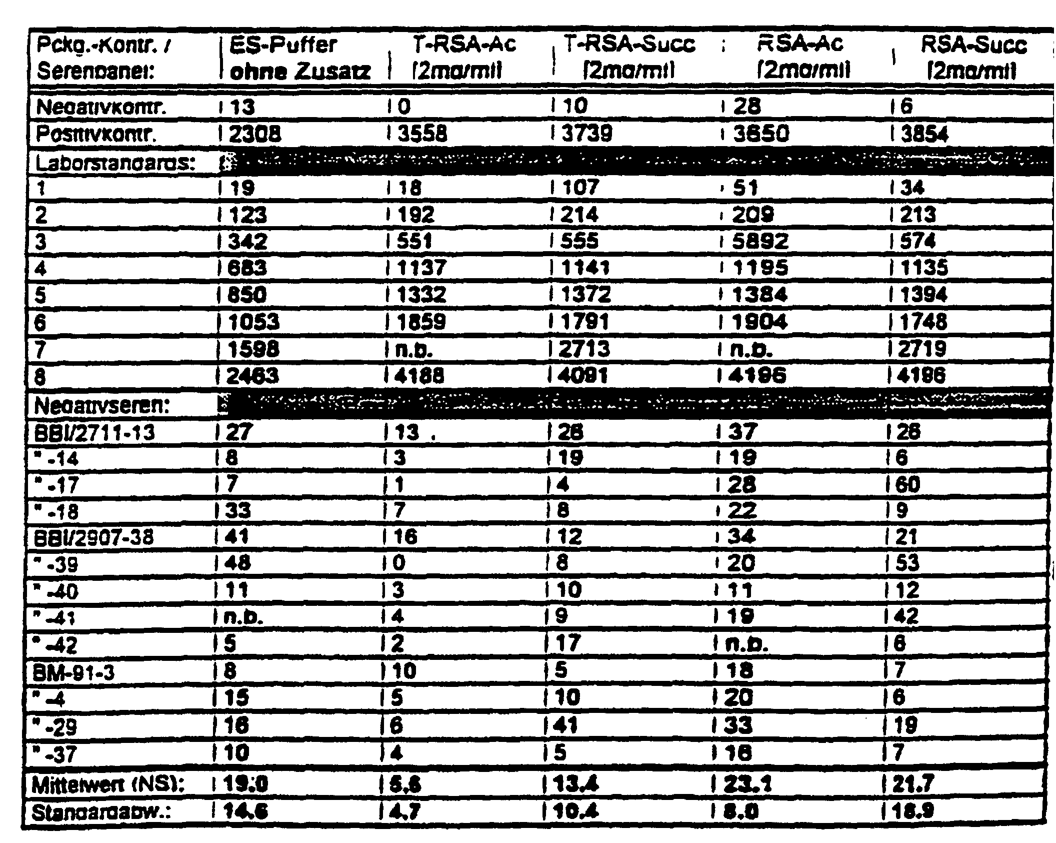

### Beispiel 2:

### Absenkung des Leerwerite im Anti-HIV-P24-Test

Ein Sandwichimmunoassay wird mit dem Testsystem Anti-HIV Enzymmuntest der Firma Boehringer Mannheim durchgeführt.

### Inkubationspuffer mit Konjugat:

Phosphatpuffer 40 mmol / l; pH 7,0
Rinderserumbestandteile HIV-P24-Antikörper biotinyliert (300 ng / ml)
Polyklonaler Anti-HIV-Antikörper POD-markiert (100 mU / ml)
Acetyliertes Thermo-RSA Partikelgröße 30 nm, 2 mg / ml in Spalte 2 von Tabelle 2

### Substratpuffer:

Phosphat/Citrat 50 mmol / l; pH 4,4
H₂O₂: 1,6 mmol / l
ABTS: 0,9 mmol / l

200 µl Probe werden mit 500 µl Inkubationspuffer 4 Stunden lang in einem Streptavidin-Tube inkubiert. Die Probenseren enthalten keine HIV-P24-Antigene. Anschließend wird gewaschen und mit 700 µl Substratpuffer I Stunde inkubiert. Es wird bei 422 nm gemessen. Die Ergebnisse zeigt Tabelle 2:

Spalte 1 gibt die gemessenen falsch-positiven Werte (in µg / ml) ohne Zusatz von acetyliertem Thermo-RSA im Inkubationspuffer an, Spalte 2 gibt die gemessenen Werte mit acetyliertem Thermo-RSA im Inkubationspuffer an. Spalte 2 zeigt, daß durch die erfindungsgemäße Störsubstanz eine Reduktion falsch-positive Signale bis zu 64% erfolgt.

| | Ohne Zus. | mit Zusarz |
|---|---|---|
| Proben | pg/ml | pg/ml |
| Kontr.Ser. | 52.25 | 51.87 |
| Kassel 4 | 13.64 | 2.66 |
| Kassel 5 | 12.32 | 3.99 |
| Kassel 6 | 9.36 | 6.80 |
| Kassel 10 | 18.73 | 5.47 |
| Kassel 11 | 18.89 | 7.68 |
| Kassel 13 | 12.65 | 2.81 |
| Kassel 16 | 12.65 | 2.81 |
| Kassel 18 | 20.87 | 12.56 |
| Satzbura 27 | 20.17 | 0.74 |
| 33 | 9.32 | 2.66 |
| 44 | 10.53 | 0.00 |
| 72 | 12.64 | 0.00 |
| 92 | 12.79 | 0.00 |
| 97 | 9.63 | 0.00 |
| 130 | 11.73 | 3.55 |
| 151 | 28.22 | 12.71 |
| 154 | 36.16 | 17.59 |
| 171 | 13.75 | 4.43 |
| 188 | 62.77 | 28.52 |
| 194 | 8.46 | 0.30 |
| 200 | 55.92 | 26.45 |
| 214 | 19.65 | 6.65 |
| 220 | 56.05 | 24.24 |
| 251 | 24.92 | 14.33 |
| 252 | 12.83 | 3.40 |
| 293 | 21.82 | 11.23 |
| 300 | 8.81 | 1.03 |
| 354 | 31.59 | 9.90 |
| 465 | 44.43 | 28.52 |
| 496 | 8.17 | 0.15 |
| 512 | 17.00 | 3.99 |
| False fos | 25 | 9 |

### Beispiel 3

Entstörung eines PSA (Prostataspezifisches Antigen) Immunoassays durch acetyliertes Thermo-RSA im Vergleich zur Entstörung durch Proteinhydrolysate (Pepton) und spezifische Entstörproteine gemäß dem Stand der Technik.

Der Immunoassay wird gemäß dem Enzymmuntest ® PSA II der Firma Boehringer Mannheim GmbH durchgeführt.
1.
   Inkubationspuffer mit Konjugat:
      Phosphatpuffer 40 mmol / l, pH 7.3
      MAK<PSA>Maus-PR12-Fab-POD 70 mU / ml
      MAK<PSA>Maus-PR1-IgG-Biotin 1 ng / ml

   Dem Inkubationspuffer werden verschiedene Entstorproteine zugesetzt (Zusätze 1, 2a-c in Tabelle 3).
2.
   Substratpuffer:
      Phosphat/Citratpuffer 100 mmol / l, pH 4,4
      H₂O₂ : 3,2 mmol/l
      Chromogen ABTS: 1,9 mmol/ l

Die Durchführung des Tests erfolgt gemäß Beispiel 1 mit 50 µm Probe und 700 ml Inkubationspuffer mit Konjugat (90 min Inkubation), 200 ml Waschlösung, 700 µl Substratpuffer (30 min. Inkubation).

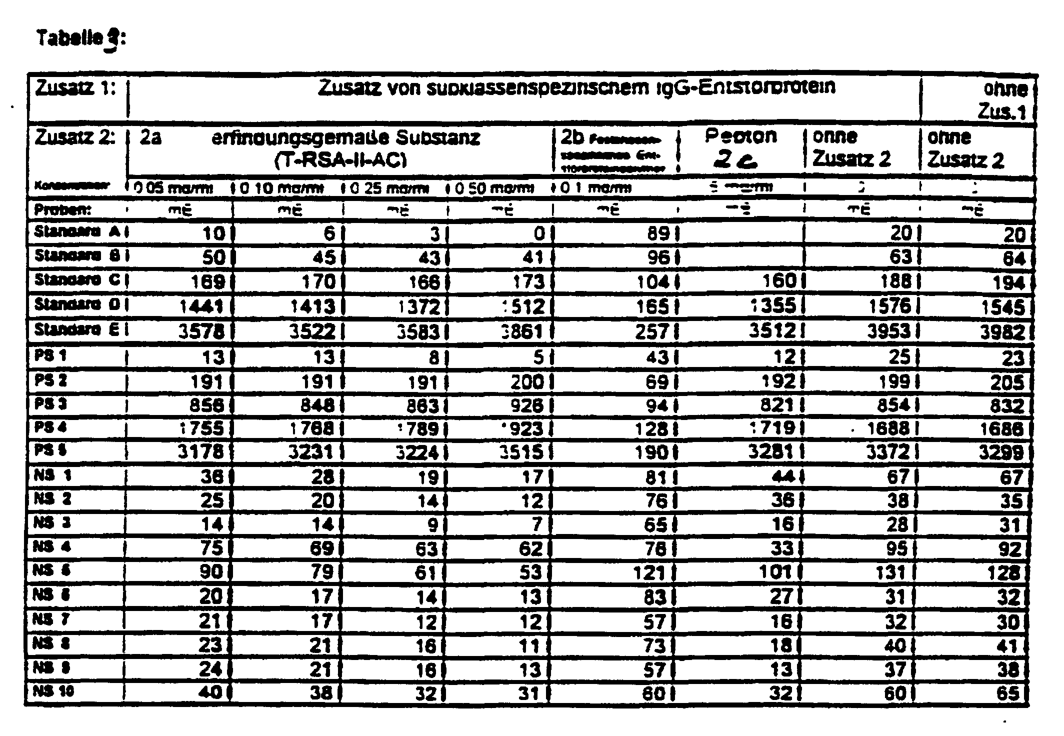

### Erläuterung zu Tabelle 3:

### PS1 bis 5:

### PSA positive Humanseren (männliche Probanten)

### NS1 - 10:

### PSA negative Humanseren (Frauenseren)

Zusatz 1 ist ein subklassenspezifisches IgG-Polymer als Entstörprotein gemäß EP-A-331 062 (Anti-PSA-Antikörperpolykonjugat), das Störkomponenten bindet. die gegen die signalgebende immunologische Komponente (Antikörper oder Antikörperfragmentkonjugat) gerichtet sind. Es zeigt in diesem Beispiel keinen Effekt (Siehe Kontrolle in der rechten Spalte "ohne Zusatz 1").

Zusatz 2a ist die erfindungsgemäße Entstörsubstanz in verschiedenen Konzentrationen (acetyliertes Thermo-RSA-Polymer, Partikelgröße 30 nm). Die Versuchsreihe zeigt, daß besonders bei einer Einsatzkonzentration größer als 0,1 mg / ml eine wirksame Entstörung der ohne Zusatz von Zusatz 2a falsch-positiv gemessen Frauenseren (NS) einsetzt. Der dynamische Messbereich wird durch Verringerung des Leerwertes (Standart A) ohne Beeinträchtigung des Signals (Steilheit der Eichkurve: Standart A - E) zudem deutlich verbessert.

Zusatz 2 b ist ein fiir Festphasen benutztes Entstörproteinpolymer in gelöster Form (Thermo-RSA). Weder in dem Tabellenbeispiel (0,1 mg/ml), noch in einem höheren Konzentrationsbereich von 0,2 bis 1,6 mg / ml zeigt dieser Inkubationspufferzusatz einen Einfluß auf die Störsignale. Mit zunehmender Konzentration an Analyt (Standart A-E) ist auch hier eine Verflachung der Eichkurve zu beobachten.

Zusatz 2 c ist ein Proteinhydrolysat (Lactalalbuminhydrolysat) gemäß Stand der Technik. Es sind hohe Konzentrationen ( 5mg / ml) für die merkliche Entstörung notwendig.

### Beispiel 4:

Das Beispiel wird wie Beispiel 3 durchgeführt, jedoch wird im Inkubationspuffer kein Zusatz (Kontrolle 1 in Tabelle 4) bzw. succinyliertes Thermo-RSA verschiedener Partikelgrößen zwischen 8 nm und 74 nm Durchmesser in einer Konzentration von 0,35 mg / ml zugesetzt.

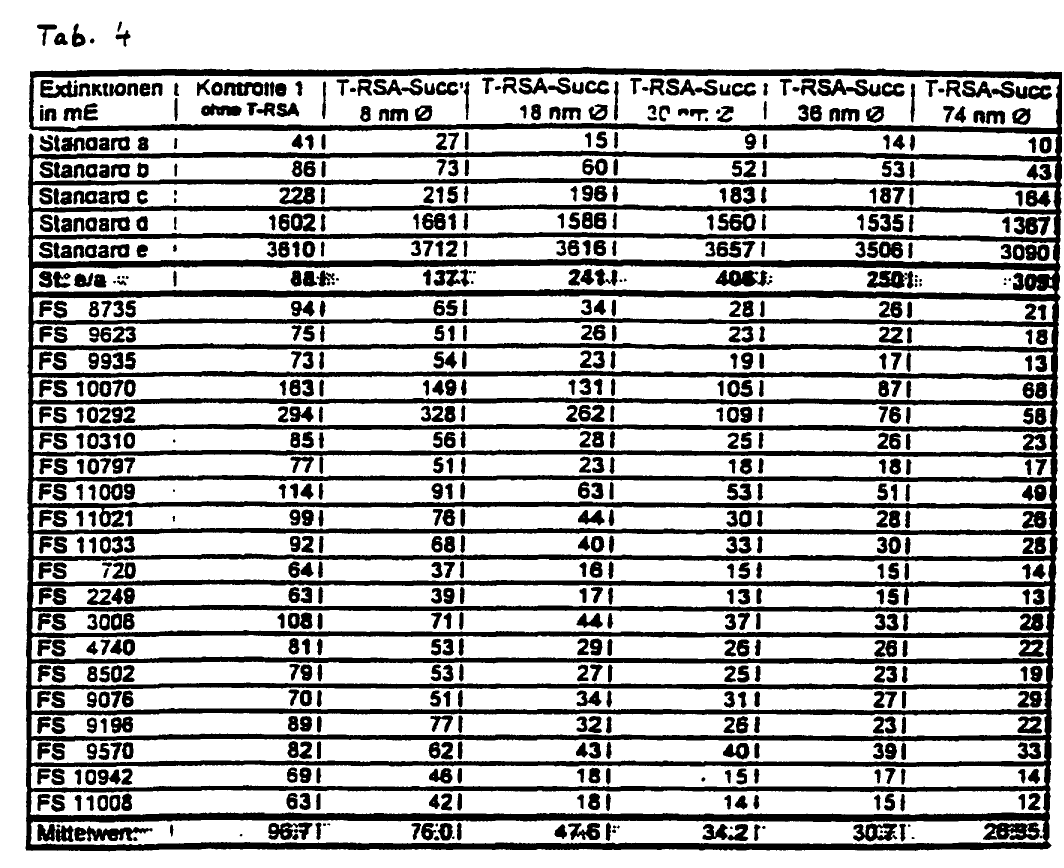

### Beispiel 5

### Herstellung von acetyliertem thermisch aggregiertem Rinderserumalbumin (acetyliertes Thermo-RSA)

### 1.

### Herstellung von thermovernetztem RSA

1 g RSA wird in 100 ml 50 mM Kaliumphosphatpufferlösung (pH 7,0) auf 70°C erwärmt und bei dieser Temperatur für 4 Stunden gehalten. Dann wird die Lösung gekühlt, filtriert und in einer Ultrazentrifuge (Ausschlußgrenze: 30.000 Da) auf eine Konzentration von 50 mg / ml gebracht. Dann wird gegen das 30-fache Volumen doppelt destilliertes Wasser dialysiert und anschließend lyophilisiert. Das Produkt hat ein Molekulargewicht von ca 700. 000 und eine Partikelgröße von 30 +/- 8 nm (gemessen über Photon Correlation Spectroscopy (PCS)).

### 2.

### Acetylierung von Thermo-RSA

4000 g thermisch aggregiertes RSA in 100 mM Kaliumphosphatpuffer pH 8,0 wird auf 25°C temperiert. Die Proteinkonzentration wird mittels OD 280 nm bestimmt und danach eine Proteinkonzentration von 10 mg / ml eingestellt. Gegebenenfalls wird mit 10 mM Kaliumphosphatpuffer pH 8,0 korrigiert. Die Thermo-RSA-Lösung im Rührkessel wird auf 25° C erwärmt. Essigsäure-N-Hydroxysuccinimid-Ester wird in einer Konzentration von 100 mg / ml in wasserfreiem DMSO bei Raumtemperatur gelöst. Pro Liter zu acetylierender Thermo-RSA-Lösung werden 11,5 ml Succinimidester-Lösung zugegeben. Die damit erreichte Endkonzentration im DMSO beträgt ca. 1%. Der Acetylierungsansatz wird dann nach Kontrolle des pH-Wertes (Soll 6,5 - 9) bei 25° C für 120 min gerührt. Die Abnahme des Essigsäure-N-Hydroxysuccinimid-Esters wird über TSK 3000 / HPLC (Detektion 260 nm) verfolgt. Nach der Inkubation wird die Acetylierung durch Zugabe von Lysinhydrochloridlösung auf 5 mM Endkonzentration gestoppt.

Der gestoppte Acetylierungsansatz wird über eine Filterpresse filtriert. Die Presse wird mit Wasser nachgewaschen. Filtrat und Nachwaschwert werden vereinigt.

Das vereinigte Filtrat wird über eine Polysulfonmembran 10 KD auf 50 I konzentriert. Die konzentrierte Lösung wird gegen das 10-fache Volumen 20 mM Kaliumphosphatlösung pH 7,0 diafiltriert. Das Konzentrat wird auf das jeweilige doppelte Volumen mit Diafiltrationspuffer verdünnt und dann wieder auf das Ausgangsvolumen konzentriert. Der Erfolg der Diafiltration wird über TSK 3000 HPLC Analytik ermittelt. Die Lösung wird auf eine Konzentration von 80 +- 10 mg / ml konzentriert und im Anschluß daran mit 0,1 % Chloracetamid und 0,01% (Methylisothiazolon) MIT stabilisiert.
Die PCS Messung liefert eine Partikelgröße von 30 nm +- 15.

### Beispiel 6

### Herstellung von chemisch polymerisiertem, succinyliertem Rinderserumalbumin (P-RSA-Succ)

### 1.Vernetzung von Rinderserumalbumin (RSA)

### a. Aktivierung von RSA mit Maleinimidohexanoyl-N-Hydroxysuccinimid (MHS)

3 g RSA werden in 30 ml 30mM Kaliumphosphat-Puffer, pH 7.1, gelöst und mit 0.6 ml einer Lösung aus 180 mg MHS / ml Dimethylsulfoxid (DMSO) versetzt. Nach 1 Std. Inkubation bei 25°C wird der Ansatz ad 10 mM Lysin aufgestockt und gegen das 150-fache Volumen Dialysepuffer (15 mM Kaliumphosphat-Puffer / 50 mM NaCl / 1 mM Ethylendiamintetraacetat (EDTA)/ pH 6.2) dialysiert.

### b. Aktivierung von RSA mit S-Acetylthiopropionyl-N-Hydroxysuccinimid (SATP)

3 g RSA werden in 30 ml 30mM Kaliumphosphat-Puffer. pH 7.1, gelöst und mit 0.6 ml einer Lösung aus 140 mg SATP / ml DMSO versetzt. Nach 1 Std. Inkubation bei 25 °C wird der Ansatz ad 10 mM Lysin aufgestockt und gegen das 150-fache Volumen Dialysepuffer (15 mM Kaliumphosphat-Puffer / 50 mM NaCl / 1 mM EDTA/ pH 6.2) dialysiert.

### c. Vernetzung der aktivierten RSA-Komponenten

Die Lösung mit dem SATP-aktivierten RSA aus (b) wird ad 25 mM Hydroxylamin aufgestockt, ein pH von 7.5 eingestellt und 1 Std. bei 25°C inkubiert. Anschließend wird die Lösung mit dem MHS-aktivierten RSA aus (a) zugesetzt und für weitere 45 min bei 25°C inkubiert. Die Vernetzung wird durch Zugabe von 10 mM Cystein gestoppt. Nach weiteren 30 Min wird der Ansatz ad 25 mM N-Methylmaleinimid aufgestockt und gegen das 150-fache Volumen 50 mM Kaliumphosphat-Puffer / 0.15 M NaCl/ pH 7.2 dialysiert.

### 2. Succinylierung

Die dialysierte poly-RSA-Lösung aus (1c) wird mit 2.6 ml einer Lösung aus 0.1 g Bernsteinsäureanhydrid / ml DMSO versetzt. Nach Inkubation für 60 Min. bei 25°C wird der Ansatz ad 50 mM Lysin aufgestockt, gegen das 150-fache Volumen 20 mM Kaliumphosphat-Puffer, pH 6.8 dialysiert und lyophilisiert.

### Beispiel 7

### Entstörung eines Troponin-T Sandwichimmunoassays durch polymerisiertes, succinyliertes RSA (P-RSA-Succ)

Der Test wird gemäß dem Enzymuntest ® Troponin-T der Firma Boehringer Mannheim GmbH durchgeführt.
1. Inkubationspuffer mit Konjugat:
   40 mM Phpsphatpuffer pH 7.0
   MAK<Troponin-T>M-7-Fab-POD 150 mU / ml
   MAK<Troponin-T>M-11-7-IGG-Biotin 2.5 µg/ml
   0.5 mg/ml P-RSA-Succ; im Vergleichstest enthält der Inkubationspuffer kein P-RSA-Succ.
2. Substratpuffer:
   Phosphat / Citratpuffer 100 mM, pH 4.4
   H₂O₂ : 3.2 mM
   Chromogen ABTS 1.9 mM

Die Durchführung des Tests erfolgt gemäß Beispiel 1 mit 140 µl Probe und 700 µl Inkubationspuffer mit Konjugat (30 min Inkubation); 200 µl Waschlösung, 700 µl Substratpuffer (15 min Inkubation).

Ohne Zusatz von P-RSA-Succ zum Inkubationspuffer liegen die in Tabelle A aufgelisteten Störseren deutlich über dem Cut-Off Wert von 0.2 ng/ml Troponin-T. Der Zusatz von 0.5 mg/ml P-RSA-Succ drückt die unspezifischen Signale aller Seren unter den Cut-Off bzw. beseitigt die Störung teilweise vollständig. Der Zusatz des Entstörproteins hat keine signifikanten Auswirkungen auf die Steigung der Eichkurve (Tabelle A). Die Wiederfindung von positiven Humanseren wird deshalb nicht beeinflußt.

Monomeres, succinyliertes RSA (RSA-Succ), entsprechend dem Stand der Technik, muß in ca. 50 fach höherer Konzentration eingesetzt werden, um annähernd die gleiche Entstörwirkung wie polymeres, succinyliertes RSA (P-RSA-Succ) zu haben (Tabelle B). Dies hat eine starke Verringerung der Steigung der Eichkurve zur Folge, was zu stark erhöhten Wiederfindungen von positiven Humanseren führt (Tabelle B). Monomeres RSA-Succ kann deshalb im Troponin-T Test nicht eingesetzt werden.

**Tabelle A:**

| **Entstörung eines Troponin-T Immunoassays durch P-RSA-Succ** | | | | |
|---|---|---|---|---|
| Cut-off : 0.2 ng/ml | | | | |
| **Störserum** | **ohne P-RSA-Succ** | | **mit P-RSA-Succ (0.5 mg/ml)** | |
| | mE | **ng/ml** | mE | **ng/ml** |
| **H 93234** | 90 | **0,392** | 44 | **0,196** |
| **R 47015** | 98 | **0,432** | 27 | **0,063** |
| **U 05706** | 106 | **0,474** | 21 | **0,003** |
| **E 02179** | 95 | **0,414** | 28 | **0,071** |
| **N 26670** | 90 | **0,39** | 20 | **<0** |
| **X 91467** | 104 | **0,462** | 37 | **0,138** |
| **R 47004** | 71 | **0,289** | 19 | **<0** |
| **X 91325** | 129 | **0,596** | 10 | **<0** |
| **G 2943** | 90 | **0,388** | 39 | **0,154** |

| **Eichkurven:** | | | |
|---|---|---|---|
| **Standard [ng/ml]** | | **ohne P-RSA-Succ mE** | **mit P-RSA- Succ mE** |
| **A** | 0 | 21 | 13 |
| **B** | 0,25 | 63 | 54 |
| **C** | 0,77 | 167 | 145 |
| **D** | 4,41 | 857 | 815 |
| **E** | 9,84 | 1921 | 1853 |
| **F** | 15,4 | 2865 | 2769 |

**Tabelle B:**

| Entstörung eines Troponin-T Immunoassays durch monomeres RSA-Succ Cut-off: 0.2 ng / ml | | | | |
|---|---|---|---|---|
| **Störseren** | **Referenz ohne RSA succ.** | **+ 10 mg/ml RSA succ.** | **+ 25 mg/ml RSA succ.** | **+ 50 mg/ml RSA succ.** |
| | **ng/ml** | **ng/ml** | **ng/ml** | **ng/ml** |
| 11033 | 0,277 | 0,088 | 0,000 | 0,048 |
| 10292 | 0,433 | 0,195 | 0,147 | 0,129 |
| 4753 | 0,223 | 0,000 | 0,000 | 0,000 |
| 10912 | 0,343 | 0,145 | 0,000 | 0,104 |

| | **Ohne RSA succ.** | **+ 25 mg/ml RSA succ.** | **% Abweichung** |
|---|---|---|---|
| **Eichkurve** | **mE** | **mE** | |
| Standard a | 16 | 4 | 25% |
| Standard b | 44 | 25 | 57% |
| Standard c | 162 | 98 | 60% |
| Standard d | 956 | 556 | 58% |
| Standard e | 1954 | 1168 | 60% |
| Standard f | 3279 | 2044 | 62% |
| | | | |

| **Positive Humanseren** | **ng/ml** | **ng/ml** | |
|---|---|---|---|
| Panel 1 | 1.031 | 1.334 | 129% |
| Panel 2 | 1.749 | 2.599 | 149% |
| Panel 3 | 3.915 | 6.223 | 159% |
| Panel 4 | 6.000 | 9.950 | 166% |
| Panel 5 | 9.047 | 14.571 | 161% |

## Patentansprüche

1. Proteinhaltige Entstörsubstanz zur Verminderung unspezifischer Wechselwirkung bei Immunoassays **dadurch gekennzeichnet, daß** die Entstörsubstanz Proteinaggregatpartikel einer bestimmten möglichst einheitlichen Größe darstellen, die mit -CO-R Gruppen acyliert sind, wobei R einen verzweigten oder unverzweigten C1-C4 Alkylrest darstellt, der mit Carboxy, Hydroxy, SO₃H oder PO₃H₂ substituiert sein kann.

2. Entstörsubstanz gemäß Anspruch 1, **dadurch gekennzeichnet, daß** das Protein ein Albumin ist.

3. Entstörsubstanz gemäß Anspruch 2, **dadurch gekennzeichnet, daß** das Albumin Rinderserumalbumin ist.

4. Entstörsubstanz gemäß den Ansprüchen 1-3, **dadurch gekennzeichnet, daß** R eine Methylgruppe oder eine -CH₂CH₂-COOH-Gruppe darstellt.

5. Entstörsubstanz gemäß einem der Ansprüche 1-4, **dadurch gekennzeichnet, daß** das Proteinaggregat chemisch mit homo- oder heterobifunktionalen Linkern aggregiert ist.

6. Entstörsubstanz gemäß einem der Ansprüche 1-4, **dadurch gekennzeichnet, daß** das Proteinaggregat thermisch aggregiert ist.

7. Entstörsubstanz gemäß Anspruch 6, **dadurch gekennzeichnet, daß** die Entstörsubstanz thermisch aggregiertes, acetyliertes oder succinyliertes Rinderserumalbumin darstellt.

8. Entstörsubstanz gemäß einem der Ansprüche 1-7, **dadurch gekennzeichnet, daß** die Paztikelgröße des acylierten Proteinaggregates 10-200 nm beträgt.

9. Entstörsubstanz gemäß Anspruch 8, **dadurch gekennzeichnet, daß** die Partikelgröße 20-50 nm beträgt.

10. Entstörmittel zur Verminderung unspezifischer Wechselwirkungen in Immunoassays enthaltend einen Puffer und eine proteinhaltige Entstörsubstanz, **dadurch gekennzeichnet, daß** es eine Entstörsubstanz gemäß einem der Ansprüche 1-9 enthält.

11. Entstörmittel gemäß Anspruch 10, **dadurch gekennzeichnet, daß** der Puffer einen pH-Wert zwischen 4 und 9 hat.

12. Spezifisches Bindungsreagenz für Immunoassays enthaltend einen spezifischen Bindungspartner eines spezifischen Bindungspaares und eine proteinhaltige Entstörsubstanz oder Entstörmittel, **dadurch gekennzeichnet, daß** es eine Entstörsubstanz gemäß einem der Ansprüche 1-9 oder ein Entstörmittel gemäß einem der Ansprüche 10 oder 11 enthält.

13. Spezifisches Bindungsreagenz gemäß Anspruch 12, **dadurch gekennzeichnet, daß** der Bindungspartner ein markierter oder biotinylierter Bindungspartner ist.

14. Spezifisches Bindungsreagenz gemäß Anspruch 12 oder 13, **dadurch gekennzeichnet**, dal der Bindungspartner ein Antigen, Antikörper oder Antikörperfragment ist.

15. Spezifisches Bindungsreagenz gemäß Anspruch 14, **dadurch gekennzeichnet, daß** der Bindungspartner ein enzymmarkierter oder elektrochemilumineszenzmarkierter Antikörper oder Antikörper-Fragment ist.

16. Verfahren zur Bestimmung eines immunologischen Liganden in einer Probe unter Reduktion unspezifischer Wechselwirkungen durch
1. Kontaktieren der auf den Liganden zu untersuchenden Probe mit
a) einer oder mehreren proteinhaltigen Entstörsubstanzen, oder einem oder mehreren proteinhaltigen Entstörmitteln enthaltend eine proteinhaltige Entstörsubstanz und einen geeigneten Puffer und
b) einem oder mehreren Bindungspartnern von spezifischen Bindungspaaren, wobei mindestens ein Bindungspartner markiert ist und ein detektierbares Bindungspaar bildet
2. Messung der Anwesenheit oder Menge des markierten Bindungspaares oder des freien markierten Partners eines spezifischen Bindungspaares als Maß für die Anwesenheit oder Konzentration des Liganden in der Probe,
**dadurch gekennzeichnet, daß** die Entstörsubstanz ein acyliertes Proteinaggregat gemäß einem der Ansprüche 1-9 ist.

17. Verfahren gemäß Anspruch 16, **dadurch gekennzeichnet, daß** der Ligand ein Virus, Virusmarker, Tumormarker oder ein Hormon darstellt.

18. Verfahren gemäß den Ansprüchen 16 oder 17, **dadurch gekennzeichnet, daß** die Bindungspartner von spezifischen Bindungspaaren aus der Gruppe der Antigene, Antikörper oder Antikörperfragmente gewählt werden.

19. Verfahren gemäß Anspruch 16 - 18, **dadurch gekennzeichnet, daß** ein Bindungspartner enzymmarkiert oder elektrochemilumineszierend ist.

20. Verfahren gemäß einem der Ansprüche 16-19, **dadurch gekennzeichnet, daß** ein spezifischer Bindungspartner eine weitere spezifische Bindungsstelle fiir einen festphasengebundenen Bindungspartner besitzt.

21. Verfahren gemäß Anspruch 20, **dadurch gekennzeichnet, daß** ein Bindungspartner biotinyliert ist und fähig ist, an eine Streptavidinoberfläche zu binden.

22. Verwendung der Entstörsubstanz gemäß einem der Anspruch 1-9 oder des Entstörmittels gemäß den Ansprüchen 10 oder 11 fiir Immunoassays.

23. Verwendung der Entstörsubstanz gemäß einem der Ansprüche 1-9 oder des Entstörmittels gemäß einem der Ansprüche 10 oder 11 zur Verminderung unspezifischer Wechselwirkungen in Immunoassays.

24. Verfahren zur Herstellung einer Entstorsubstanz gemäß Anspruch 1, **dadurch gekennzeichnet, daß** a) Protein zu einem Aggregat polymerisiert wird b) daß das Proteinaggregat mit einem entsprechenden Acylierungsmittel acyliert wird.

25. Verfahren gemäß Anspruch 24, **dadurch gekennzeichnet, daß** die Polymerisierung des Proteins thermisch erfolgt.

26. Verfahren gemäß Anspruch 24, **dadurch gekennzeichnet, daß** das Proteinaggregat mit Acetyl-O-Succinimid acetyliert oder mit Bernsteinsäureanhydrid succinyliert wird.

## Claims

1. Protein-containing interference-reducing substance for reducing unspecific interactions in immunoassays **characterized in that** the interference-reducing substance consists of protein aggregate particles of a certain size which is as uniform as possible and the said particles are acylated with -CO-R groups in which R represents a branched or unbranched C1-C4 alkyl residue which can be substituted with carboxy, hydroxy, SO₃H or PO₃H₂.

2. Interference-reducing substance as claimed in claim 1, **characterized in that** the protein is an albumin.

3. Interference-reducing substance as claimed in claim 2, **characterized in that** albumin is bovine serum albumin.

4. Interference-reducing substance as claimed in claims 1-3, **characterized in that** R represents a methyl group or a -CH₂CH₂-COOH group.

5. Interference-reducing substance as claimed in one of the claims 1-4, **characterized in that** the protein aggregate is chemically aggregated with homo- or heterobifunctional linkers.

6. Interference-reducing substance as claimed in one of the claims 1-4, **characterized in that** the protein aggregate is thermally aggregated.

7. Interference-reducing substance as claimed in claim 6, **characterized in that** the interference-reducing substance is thermally aggregated, acetylated or succinylated bovine serum albumin.

8. Interference-reducing substance as claimed in one of the claims 1-7, **characterized in that** the particle size of the acylated protein aggregate is 10-200 nm.

9. Interference-reducing substance as claimed in claim 8, **characterized in that** the particle size is between 20 and 50 nm.

10. Interference-reducing agent for reducing unspecific interactions in immunoassays containing a buffer and a protein-containing interference-reducing substance, **characterized in that** it contains an interference-reducing substance as claimed in one of the claims 1-9.

11. Interference-reducing agent as claimed in claim 10, **characterized in that** the buffer has a pH between 4 and 9.

12. Specific binding reagent for immunoassays containing a specific binding partner of a specific binding pair and a protein-containing interference-reducing substance or interference-reducing agent, **characterized in that** it contains an interference-reducing substance according to one of the claims 1-9 or an interference-reducing agent according to one of the claims 10 or 11.

13. Specific binding reagent as claimed in claim 12, **characterized in that** the binding partner is a labelled or biotinylated binding partner.

14. Specific binding reagent as claimed in claim 12 or 13, **characterized in that** the binding partner is an antigen, antibody or antibody fragment.

15. Specific binding reagent as claimed in claim 14, **characterized in that** the binding partner is an enzyme-labelled or electrochemiluminescence-labelled antibody or antibody fragment.

16. Method for determining an immunological ligand in a sample while reducing unspecific interactions by
1. contacting the sample to be examined for the ligand with
a) one or more protein-containing interference-reducing substances or one or more protein-containing interference-reducing agents containing a protein-containing interference-reducing substance and a suitable buffer and
b) one or more binding partners of specific binding pairs wherein at least one binding partner is labelled and forms a detectable binding pair
2. measuring the presence or amount of the labelled binding partner or of the free labelled partner of a specific binding pair as a measure for the presence or concentration of the ligand in the sample,
**characterized in that** the interference-reducing substance is an acylated protein aggregate as claimed in one of the claims 1-9.

17. Method as claimed in claim 16, **characterized in that** the ligand is a virus, virus marker, tumour marker or a hormone.

18. Method as claimed in claims 16 or 17, **characterized in that** the binding partners of specific binding pairs are selected from the group comprising antigens, antibodies or antibody fragments.

19. Method as claimed in claim 16 - 18, **characterized in that** one binding partner is enzyme-labelled or electrochemiluminescent.

20. Method as claimed in one of the claims 16 - 19, **characterized in that** one specific binding partner has another specific binding site for a binding partner bound to a solid phase.

21. Method as claimed in claim 20, **characterized in that** one binding partner is biotinylated and is able to bind to a streptavidin surface.

22. Use of the interference-reducing substance as claimed in one of the claims 1-9 or of the interference-reducing agent as claimed in claim 10 or 11 for immunoassays.

23. Use of the interference-reducing substance as claimed in one of the claims 1-9 or of the interference-reducing agent as claimed in one of the claims 10 or 11 to reduce unspecific interactions in immunoassays.

24. Method for preparing an interference-reducing substance as claimed in claim 1, **characterized in that** a) a protein is polymerized to form an aggregate, b) the protein aggregate is acylated with an appropriate acylation agent.

25. Method as claimed in claim 24, **characterized in that** the protein is thermally polymerized.

26. Method as claimed in claim 24, **characterized in that** the protein aggregate is acetylated with acetyl-O-succinimide or succinylated with succinic anhydride.

## Revendications

1. Substance protéinique réductrice des interférences pour réduire une interaction non spécifique dans les immunodosages, **caractérisée en ce que** la substance réductrice des interférences représente des particules d'agrégat protéinique d'une taille déterminée si possible uniforme, qui sont acylées par des groupes -CO-R où R représente un résidu alkyle en C₁ à C₄ ramifié ou non ramifié, qui peut être substitué par un groupe carboxy, hydroxy, SO₃H ou PO₃H₂.

2. Substance réductrice des interférences selon la revendication 1, **caractérisée en ce que** la protéine est une albumine.

3. Substance réductrice des interférences selon la revendication 2, **caractérisée en ce que** l'albumine est une albumine de sérum bovin.

4. Substance réductrice des interférences selon les revendications 1 à 3, **caractérisée en ce que** R représente un groupe méthyle ou un groupe -CH₂CH₂-COOH.

5. Substance réductrice des interférences selon l'une quelconque des revendications 1 à 4, **caractérisée en ce que** l'agrégat protéinique est agrégé chimiquement avec des lieurs homo- ou hétéro-bifonctionnels.

6. Substance réductrice des interférences selon l'une quelconque des revendications 1 à 4, **caractérisée en ce que** l'agrégat protéinique est agrégé thermiquement.

7. Substance réductrice des interférences selon la revendication 6, **caractérisée en ce que** la substance réductrice des interférences représente de l'albumine de sérum bovin acétylée ou succinylée, agrégée thermiquement.

8. Substance réductrice des interférences selon l'une quelconque des revendications 1 à 7, **caractérisée en ce que** la taille de particule de l'agrégat protéinique acylé est de 10 à 200 nm.

9. Substance réductrice des interférences selon la revendication 8, **caractérisée en ce que** la taille de particule est de 20 à 50 nm.

10. Agent réducteur des interférences pour réduire les interactions non spécifiques dans des immunodosages contenant un tampon et une substance protéinique réductrice des interférences, **caractérisé en ce qu'**il contient une substance réductrice des interférences selon l'une quelconque des revendications 1 à 9.

11. Agent réducteur des interférences selon la revendication 10, **caractérisé en ce que** le tampon présente une valeur de pH comprise entre 4 et 9.

12. Réactif de liaison spécifique pour immunodosage contenant un partenaire de liaison spécifique d'une paire de liaison spécifique et une substance réductrice des interférences ou agent réducteur des interférences protéinique, **caractérisé en ce qu'**il contient une substance réductrice des interférences selon l'une quelconque des revendications 1 à 9 ou un agent réducteur des interférences selon l'une quelconque des revendications 10 ou 11.

13. Réactif de liaison spécifique selon la revendication 12, **caractérisé en ce que** le partenaire de liaison est un partenaire de liaison marqué ou biotinylé.

14. Réactif de liaison spécifique selon la revendication 12 ou 13, **caractérisé en ce que** le partenaire de liaison est un antigène, un anticorps ou un fragment d'anticorps.

15. Réactif de liaison spécifique selon la revendication 14, **caractérisé en ce que** le partenaire de liaison est un anticorps ou un fragment d'anticorps marqué par un enzyme ou marqué par électrochimioluminescence.

16. Procédé de détermination d'un ligand immunologique dans un échantillon avec réduction des interactions non spécifiques par
1. la mise en contact de l'échantillon à analyser du point de vue du ligand avec
a) une ou plusieurs substances protéiniques réductrices des interférences, ou un ou plusieurs agents protéiniques réducteurs des interférences contenant une substance protéinique réductrice des interférences et un tampon approprié et
b) un ou plusieurs partenaires de liaison de paires de liaison spécifiques, au moins un partenaire de liaison étant marqué et formant une paire de liaison détectable,
2. la mesure de la présence ou de la quantité de la paire de liaison marquée ou du partenaire marqué libre d'une paire de liaison spécifique en tant que mesure de la présence ou de la concentration du ligand dans l'échantillon,
**caractérisé en ce que**, la substance réductrice des interférences est un agrégat protéinique acylé selon l'une quelconque des revendications 1 à 9.

17. Procédé selon la revendication 16, **caractérisé en ce que** le ligand représente un virus, un marqueur de virus, un marqueur de tumeur ou une hormone.

18. Procédé selon les revendications 16 ou 17, **caractérisé en ce que** les partenaires de liaison de paires de liaison spécifiques sont choisis dans le groupe des antigènes, des anticorps ou des fragments d'anticorps.

19. Procédé selon les revendications 16 à 18, **caractérisé en ce qu'**un partenaire de liaison est marqué par un enzyme ou est électrochimioluminescent.

20. Procédé selon l'une quelconque des revendications 16 à 19, **caractérisé en ce qu'**un partenaire de liaison spécifique possède un site de liaison spécifique supplémentaire pour un partenaire de liaison lié à une phase solide.

21. Procédé selon la revendication 20, **caractérisé en ce qu'**un partenaire de liaison est biotinylé et capable de se lier à une surface de streptavidine.

22. Utilisation de la substance réductrice des interférences selon l'une quelconque des revendications 1 à 9 ou de l'agent réducteur des interférences selon les revendications 10 ou 11 pour des immunodosages.

23. Utilisation de la substance réductrice des interférences selon l'une quelconque des revendications 1 à 9 ou de l'agent réducteur des interférences selon l'une quelconque des revendications 10 ou 11 pour réduire les interactions non spécifiques dans les immunodosages.

24. Procédé de préparation d'une substance réductrice des interférences selon la revendication 1, **caractérisé en ce que** a) une protéine est polymérisée en un agrégat, b) l'agrégat protéinique est acylé par un agent d'acylation approprié.

25. Procédé selon la revendication 24, **caractérisé en ce que** la polymérisation de la protéine a lieu thermiquement.

26. Procédé selon la revendication 24, **caractérisé en ce que** l'agrégat protéinique est acétylé par de l'acétyl-O-succinimide ou succinylé par de l'anhydride succinique.
